# EUROPEAN PATENT APPLICATION

(11) **EP 4 331 717 A1**
(43) Date of publication of application: **06.03.2024**
(21) Application number: 22193480.5
(22) Date of filing: 01.09.2022
(51) Int. Cl.: B01J 3/02, B01J 8/00, C01C 1/04, C07C 273/04, C07C 273/16

(54) **METHOD FOR REDUCING CARBON DIOXIDE EMISSIONS IN A UREA PLANT**

(71) Applicant: Yara International ASA, 0277 Oslo (NO)
(72) Inventor: Porro, Lino Giovanni, 1040 Etterbeek (BE); Versteele, Wim, 9961 Boekhoute (BE)
(74) Representative: De Clercq & Partners

(57) **Abstract**

The present disclosure provides a urea-producing plant comprising a first steam network, configured to receive high-pressure steam, and a second steam network, configured to receive low-pressure steam, wherein the plant comprises a steam compressor configured to receive steam from the second steam network, to convert low-pressure steam into high-pressure steam and to deliver the high-pressure steam to the first steam network. The present disclosure provides a method for reducing carbon dioxide emissions from a urea-producing plant.

## Description

### Field of the invention

The present disclosure is related to the field of urea production.

### Background of the invention

Urea (H₂NCONH₂) is the most common nitrogen-containing fertilizer used today. Global annual production is estimated to be around 200 million tons. In addition to the use as fertilizer, urea is also used in various chemical synthesis, and as a solution in diesel-powered vehicles to reduce nitrogen oxides emissions.

Urea-producing plants may use different technologies, such as the Stamicarbon or Saipem process, but they all rely on the same basic principles. In the synthesis section of a urea-producing plant, ammonia and carbon dioxide (CO₂) are reacted under high pressure (above 130 bar) and high temperatures, to produce an aqueous solution comprising urea, ammonium carbamate, and free ammonia. This aqueous solution is then purified and concentrated to obtain a urea melt. The urea melt can be diluted to obtain a urea aqueous solution which may be used in selective catalytic or noncatalytic reduction systems, such as diesel exhaust fluid, mixed with other nutrient sources, such as ammonium nitrate, to obtain aqueous solutions of urea ammonium nitrate, or solidified into ureacontaining solids, such as granules or prills.

The production of urea requires the input of high amounts of energy, in particular due to the reaction equilibrium existing between the starting materials and the final product. In most urea plants, this energy is obtained by burning non-renewable energy sources, such as natural gas or oil, to heat up water and produce steam. However, this production of energy releases vast amounts of carbon dioxide, a greenhouse gas, into the atmosphere, and there is a constant requirement from authorities and clients to reduce emissions of carbon dioxide from urea production.

### Summary of the invention

The present inventors have identified a new method to reduce carbon dioxide emissions in a urea plant and have designed a urea plant with reduced carbon dioxide emissions.

In a first aspect, the present disclosure provides a urea-producing plant comprising a first steam network, configured to receive high-pressure steam, and a second steam network, configured to receive low-pressure steam, wherein the plant comprises a steam compressor configured to receive steam from the second steam network, to convert low-pressure steam into high-pressure steam and to deliver the high-pressure steam to the first steam network.

In another aspect, the present disclosure provides a method for reducing carbon dioxide emissions from a urea-producing plant according to the present disclosure, comprising the steps of:
- providing low-pressure steam from the second steam network to the steam compressor;
- converting the low-pressure steam into high pressure steam in the steam compressor;
- directing this high-pressure steam to the first steam network.

### Brief description of the figures

The following description of the figure of a specific embodiment of a system according to the present disclosure is only given by way of example and is not intended to limit the present explanation, its application or use. In the figure, identical reference numerals refer to the same or similar parts and features.
Figure 1 represents an embodiment of a urea plant according to the present disclosure.
Figure 2 represents another embodiment of a urea plant according to the present disclosure.

### Detailed description of the invention

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

All references cited in this description are hereby deemed to be incorporated in their entirety by way of reference.

As used herein, the following terms have the following meanings:
"A", "an", and "the" as used herein refers to both singular and plural referents unless the context clearly dictates otherwise. By way of example, "a device" refers to one or more than one device.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

A "urea plant" means a plant suitable for producing urea. The final product of the plant may be solid or liquid.

In a first aspect, the present disclosure provides a urea-producing plant comprising a first steam network, configured to receive high-pressure steam, and a second steam network, configured to receive low-pressure steam, wherein the plant comprises a steam compressor configured to receive steam from the second steam network, to convert low-pressure steam into high-pressure steam and to deliver the high-pressure steam to the first steam network.

It was found out that it was possible to reduce carbon dioxide emissions by transforming useless low-pressure steam produced by one or more devices of the urea plant into valuable high-pressure steam, that can be re-used in the plant, using a steam compressor.

As stated above, several steps of urea production are endothermic and thus require an input of energy. This energy is usually provided in the form of steam, superheated or saturated, which can be at different pressures.

A urea-producing plant comprises at least two steam networks comprising steam at different pressures: a high-pressure (HP) steam network, also referred to herein and used interchangeably herein with the term "a first steam network", configured to receive steam at a (high) pressure of at least 1.5 MPa, and a low-pressure (LP) steam network, also referred to herein and used interchangeably herein with the term "a second steam network", configured to receive steam at a (low) pressure of from 0.1 to 0.7 MPa

High-pressure steam is conventionally produced by boilers, which burn fossil fuels, such as natural gas or oil, and thus produce carbon dioxide. Alternatively, high-pressure steam can also be obtained from a steam turbine as steam extraction, if the plant comprises a steam turbine, for example to drive a CO₂ compressor. However, this requires to produce steam with an even higher pressure, thus increasing the release of carbon dioxide from the boilers. So, a way to reduce carbon dioxide emissions of a urea plant is to reduce the amount of steam that a plant requires to produce for its operation.

A urea plant comprises many devices that require steam to operate, such as evaporators, strippers, carbamate decomposers, desorbers, steam ejectors and hydrolyzers. For example, some high-pressure device such as a HP stripper and a urea hydrolyzer require HP steam as input and are connected to the HP steam network. In addition, some high-pressure devices produce low-pressure steam, i.e. steam at a pressure of from 0.1 to 0.7 MPa, for example carbamate condensers operating at a pressure (process) higher than 3.0 MPa.

This low-pressure steam may be used by one or more of devices in the urea-plant. LP steam may also be injected into a steam turbine present in the plant to produce power, for example for a compressor. If there is an excess of LP steam produced by the plant and not consumed by any device, the LP steam may be released in the atmosphere, as atmospheric steam or condensed in a cooler as process condensate. In both cases, this represents a waste of energy for the plant.

However, it was found that it is possible to re-use that LP steam in the urea plant by converting it into high-pressure steam that is used by one or more HP devices in the plant, such as HP strippers and urea hydrolyzers. The conversion is done by a steam compressor connected to the both the LP and the HP steam network, wherein the steam compressor is configured to receive low-pressure steam from the second steam network, i.e. the low-pressure steam network, to convert the low-pressure steam into high-pressure steam and to deliver this high-pressure steam to the first steam network, i.e. the high-pressure steam network.

In some embodiments, the steam compressor is a two-stage compressor. It may be required to have a two-stage compressor because the pressure difference between the LP steam received by the compressor and the HP steam used by the plant may be so important that a single-stage compressor is not able to perform that transformation In particular, a two-stage compressor is required to transform LP steam at 0.5 MPa into HP steam at 2.3 MPa.

As used herein, the term "high-pressure steam", particularly related to the first steam network, generally relates to steam having a pressure of at least 1.5MPa. In some embodiments, the first or high-pressure steam network is configured to receive steam at a pressure of from 1.5 to 11 MPa, from 2.0 to 11 MPa, from 2.0 to 10 MPa, from 2.0 to 9.0 MPa, from 2.0 to 8.0 MPa, from 2.0 to 7.0 MPa, from 2.0 to 6.0 MPa, from 2.0 to 5.0 MPa, from 2.0 to 4.0 MPa, from 2.0 to 3.0 MPa, from 2.0 to 2.9 MPa, from 2.0 to 2.7 MPa, from 2.0 to 2.6 MPa, from 2.0 to 2.5 MPa, from 2.0 to 2.4 MPa, from 1.5 to 2.9 MPa, from 1.5to 2.7 MPa, from 1.5 to 2.6 MPa, from 1.5 to 2.5 MPa, or from 1.5 to 2.4 MPa, from 1.8 to 2.9 MPa, from 1.8 to 2.7 MPa, from 1.8 to 2.6 MPa, from 1.8 to 2.5 MPa, or from 1.8 to 2.4 MPa. The HP steam network may be configured to receive steam at a pressure specific to the plant. Urea plants use different technologies depending on which company built the plant. For example, in a Stamicarbon plant, i.e. a plant built by the technology provider Stamicarbon, the HP steam network is configured to receive steam at a pressure of from 1.8 to 2.5 MPa.

As used herein, the term "low-pressure steam", particularly related to the second steam network, generally relates to steam having a pressure of between 0.1 and 1.0 MPa. In some embodiments, the second or low-pressure steam network is configured to receive steam at a pressure of from 0.1 to 0.7 MPa, from 0.2 to 0.7 MPa, from 0.1 to 0.6 MPa, from 0.2 to 0.6 MPa, from 0.3 to 0.6 MPa, or from 0.3 to 0.7 MPa. The LP steam network may be configured to receive steam at a pressure specific to the plant.

In some embodiments, the high-pressure device connected to the first or high-pressure steam network is a high-pressure stripper. A HP stripper is a device configured to remove ammonium carbamate, ammonia and carbon dioxide from the aqueous solution produced by the synthesis reactor. A HP stripper uses HP steam to heat up an aqueous solution comprising urea, ammonium carbamate, and free ammonia. Upon heating the ammonium carbamate decomposes into ammonia and carbon dioxide which escape the aqueous solution as gases. The HP stripper may be connected to a condenser configured to condense the gas stream comprising water, ammonia, and carbon dioxide, produced by the HP stripper.

In some embodiments, the plant comprises a urea hydrolyzer connected to the first or high-pressure steam network. A urea hydrolyzer is a device configured to remove urea from process condensate produced in the urea plant. Process condensate refers to an aqueous solution obtained by condensing a gas stream comprising water vapor. A gas stream comprising water vapor may comprise other components such as urea, and the condensate of that gas stream contains urea as well. However, it may be preferred that process condensate does not contain urea, and a urea hydrolyzer can be used to achieve that goal. Urea hydrolyzers also use HP steam to decompose urea back into ammonia and carbon dioxide.

A urea plant comprises at least one low-pressure device connected to the LP steam network. As used herein, a low-pressure device refers to a device that requires the input of low-pressure steam to operate.

In some embodiments, a low-pressure device connected to the second or low-pressure steam network is an evaporator. Evaporators are widely used in urea plants to remove water from aqueous solutions to increase the concentration of the components comprised therein, for example to concentrate an aqueous solution comprising urea. Evaporators may use steam to heat up the aqueous solution and evaporate water from it. Evaporating water is an exothermic operation, so an evaporator receives steam at a first pressure, and produces steam condensate.

In some embodiments, a low-pressure device connected to the second or low-pressure steam network is a carbamate decomposer. A carbamate decomposer is a device configured to receive an aqueous composition comprising urea and ammonium carbamate. The carbamate decomposer is configured to heat up the aqueous composition such that the ammonium carbamate present therein decomposes into ammonia and carbon dioxide, which can be separated from the aqueous composition. A carbamate decomposer may use LP steam as heat source to heat up the aqueous composition.

In some embodiments, a low-pressure device connected to the second or low-pressure steam network is a desorber. A desorber is a device configured to remove gases, in particular ammonia and/or carbon dioxide, from aqueous solutions. A desorber receives LP steam, which is partially condensed.

In some embodiments, the device connected to the second or low-pressure steam network is a steam ejector.

The synthesis section of a urea plant requires a supply of carbon dioxide under high pressure, in particular from 13 to 16 MPa. In order to obtain this carbon dioxide under high pressure, urea plants comprise a carbon dioxide compressor. The carbon dioxide compressor requires a high amount of mechanical power which may be provided by a variety of devices, such as gas or steam turbines or electric motors.

In some embodiments, a first electric motor is connected to the carbon dioxide compressor and configured to provide power to the carbon dioxide compressor. An electric motor has the advantage that it does not generate any greenhouse gas emissions, in particular no carbon dioxide emissions, and it can be supplied with electricity generated from sources not emitting any greenhouse gases. Gas turbines use natural gas, which generates greenhouse gas emissions. Steam turbines may be an advantage in a urea plant as they are able to transform low-pressure steam into mechanical power for the compressor. However, the supply of LP steam may not be enough to generate enough power and turbines require an additional supply of HP steam which is often produced by burning fossil fuels, and thus generates carbon dioxide emissions.

In some embodiments, the plant comprises a second electric motor configured to provide power to the steam compressor. A steam compressor requires an input of mechanical power to operate. In order to not further increase the greenhouse gas emissions, it may be an advantage to find a way to provide power to the steam compressor that does not emit carbon dioxide or any other greenhouse gas. An electric motor can be considered to emit no carbon dioxide if the electricity provided to it is generated without carbon dioxide emissions, such as wind-, solar-, hydro-, or nucleargenerated electricity.

In some embodiments, the plant comprises a steam turbine configured to provide power to the steam compressor. In many cases, an industrial site comprising a urea plant also comprises an ammonia plant that produces ammonia used by the urea plant. Ammonia plants produce HP steam at 4.0 MPa, particularly in a third steam network. Since urea plants may only require HP steam at pressures from 2.0 to 3.0 MPa, the plant may comprise a system to produce HP steam at a pressure of from 2.0 to 3.0 MPa (particularly in the first steam network) from 4.0 MPa steam (particularly in a third steam network). Instead, a back-pressure steam turbine may be configured to receive high pressure steam, such as 4.0 MPa steam, produce mechanical power to supply power to the steam compressor and let out HP steam at a pressure of from 2.0 to 3.0 MPa, which is directed to the first HP steam network of the urea plant. While the presence of a steam turbine increases the steam consumption of the urea plant, and therefore its greenhouse gas emissions, compared to a urea plant wherein the steam compressor is powered by an electric motor, this configuration still represents an improvement of a conventional urea plant not comprising a steam compressor. The skilled person understands that, because the steam turbine provides HP steam to the urea plant (to the first steam network) and at the same time provides power to the steam compressor, this allows the plant to increase the LP steam pressure and to re-use its LP steam, resulting in a net gain, i.e. a decrease, in steam consumption.

In some embodiments, the plant comprises an electric motor and a steam turbine connected to the steam compressor and configured to provide power to the steam compressor. A steam turbine may be an interesting device to use if the plant has some excess steam that is not used by any device. However, the amount of excess steam available may not be enough to provide all the power required by the steam compressor. Instead of increasing the steam production, which may increase the carbon dioxide emissions of the plant, it may be an advantage to install an electric motor to provide the missing power to the steam compressor.

In some embodiments, the steam turbine is connected to the first or high-pressure steam network and configured to provide power to the steam compressor.

In another aspect, the present disclosure provides a method for reducing carbon dioxide emissions from a urea-producing plant according to the present disclosure, comprising the steps of:
- providing low-pressure steam from the second or low-pressure steam network to the steam compressor;
- converting the low-pressure steam into high pressure steam in the steam compressor;
- directing this high-pressure steam to the first or high-pressure steam network.

In some embodiments, the method comprises the steps of:
- providing low-pressure steam from the second or low-pressure steam network to the one or more low-pressure devices;
- providing low-pressure steam from the second or low-pressure steam network to the steam compressor;
- converting the low-pressure steam into high-pressure steam;
- directing the high-pressure steam produced by the steam compressor to the first or high-pressure steam network;
- providing high-pressure steam from the first or high-pressure steam network to the one or more high-pressure devices.

In another aspect, the present disclosure provides the use of a steam compressor in a urea-producing plant for reducing the carbon dioxide emissions of the urea-producing plant.

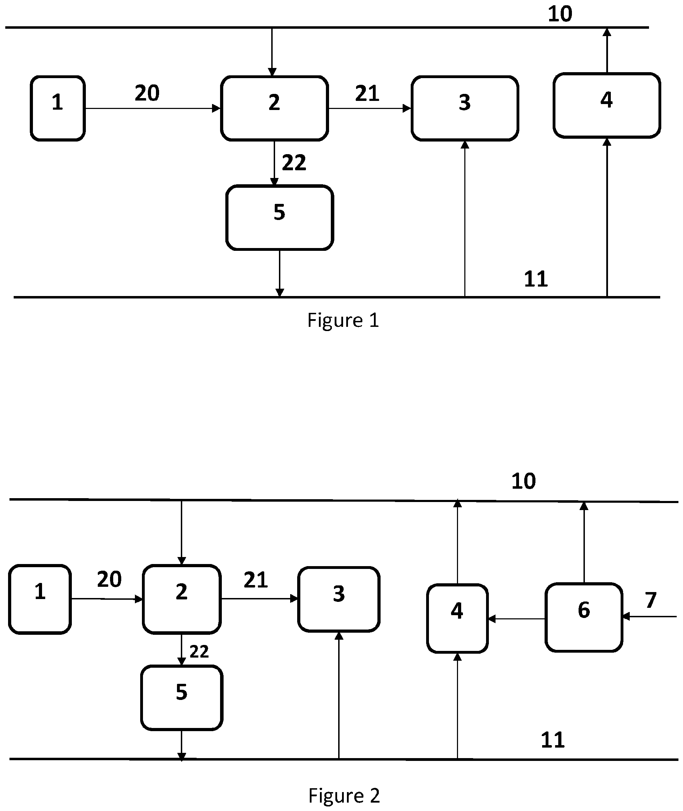

Figure 1 shows an embodiment of a urea plant according to the present disclosure. The plant comprises a synthesis reactor **1** configured to mix ammonia and carbon dioxide and to produce an aqueous solution **20** comprising urea, ammonium carbamate, and free ammonia. The synthesis section **1** is connected to HP stripper **2.** The HP stripper **2** is also connected to the HP steam network **10** configured to receive and distribute steam at a pressure of from 2.0 to 3.0 MPa. The HP stripper **2** is configured to remove some ammonium carbamate, free ammonia, and water from the aqueous solution **20** and to produce an aqueous solution **21** with less impurities and a higher urea concentration than solution **20.** The HP stripper **2** is also configured to produce a gas stream **22** comprising ammonia, carbon dioxide, and water. The HP stripper **2** is connected to a HP carbamate decomposer **5,** which is configured to receive the gas stream **22.** The HP carbamate condenser **5** is configured to condense the gas stream **22** into an aqueous solution and to produce LP steam. The HP carbamate condenser **5** is connected to the LP steam network **11,** such that the LP steam produced by the HP carbamate condenser **5** can be directed to the LP steam network **11.** The HP stripper **2** is also connected to an LP section **3.** The LP section **3** comprises one or more devices that are configured to consume LP steam, such as a carbamate decomposer, an evaporator, a desorber, and/or a steam ejector. The LP section **3** is connected to the LP steam network **11,** such that the devices of the LP section can receive LP steam. The plant comprises a steam compressor **4,** driven by an electrical motor (not shown), connected to the HP steam network **10** and the LP steam network **11,** and is configured to convert LP steam into HP steam.

Figure 2 shows another embodiment of a urea plant according to the present disclosure. Similar to the plant shown in Figure 1, the plant comprises a synthesis reactor **1,** configured to produce an aqueous solution **20** comprising urea, ammonium carbamate, and free ammonia, a HP stripper **2,** a HP carbamate decomposer **5,** and an LP section **3.** The plant comprises a steam compressor **4** connected to the HP steam network **10** and the LP steam network **11** and is configured to convert LP steam into HP steam. The plant also comprises a back-pressure steam turbine **6,** which is connected to a 4.0 MPa (or higher pressure if available) steam network (i.e. a third steam network) by the connection **7.** The steam turbine **6** is connected to the compressor **4** and configured to provide power to it. The steam turbine **6** is also connected to the HP steam network **10** and is configured to release HP steam at the same pressure as the steam comprised in the HP steam network **10.**

## Claims

1. A urea-producing plant, comprising a first steam network, configured to receive high-pressure steam, and a second steam network, configured to receive low-pressure steam **characterized in that** the plant comprises a steam compressor configured to receive steam from the second steam network, to convert low-pressure steam into high-pressure steam and to deliver the high-pressure steam to the first steam network.

2. The urea-producing plant according to claim 1, wherein the first steam network is configured to receive steam at a pressure from 1.5 to 11 MPa.

3. The urea-producing plant according to claim 1 or 2, wherein the second steam network is configured to receive steam at a pressure of from 0.1 to 0.7 MPa.

4. The urea-producing plant according to any one of claims 1 to 3, wherein the plant comprises one or more high-pressure devices connected to the first steam network, these one or more high pressure devices being a high-pressure stripper or a urea hydrolyzer.

5. The urea-producing plant according to any one of claims 1 to 4, wherein the plant comprises one or more low-pressure devices connected to the second steam network, in particular wherein these one or more low-pressure devices are selected from the group consisting of an evaporator, carbamate decomposer, desorber, steam ejector, steam turbine and carbamate condenser.

6. The urea-producing plant according to any one of claims 1 to 5, wherein the steam compressor is connected to a steam turbine, which is connected to the first steam network and configured to provide power to the steam compressor.

7. The urea-producing plant according to any one of claims 1 to 6, wherein the steam compressor is a two-stage compressor.

8. The urea-producing plant according to any one of claims 1 to 7 wherein the plant comprises a carbon dioxide compressor and a first electric motor connected to the carbon dioxide compressor and configured to provide power to the carbon dioxide compressor.

9. The urea-producing plant according to any one of claims 1 to 8, wherein the plant comprises a second electric motor which is connected to and configured to provide power to the steam compressor.

10. A method for reducing carbon-dioxide emissions of a urea-producing plant according to any one of claims 1 to 9, comprising the steps of:
- providing low-pressure steam from a second steam network to the steam compressor;
- converting the low pressure-steam into high-pressure steam by the steam compressor;
- directing the high-pressure steam produced by the steam processor to a first steam network.

11. A method according to claim 10, comprising the steps of
- providing low-pressure steam from the second steam network to one or more low-pressure devices;
- providing low-pressure steam from the second steam network to the steam compressor;
- converting the low-pressure steam into high-pressure steam;
- directing the high-pressure steam produced by the steam compressor to the first steam network;
- providing high-pressure steam from the high-pressure steam network to one or more high-pressure devices.

12. Method according to claim 10 or 11, wherein the low-pressure steam of the second steam network has a pressure from 0.1 to 0.7 MPa.

13. Method according to any one of claims 10 to 12, wherein the high-pressure steam produced by the steam processor and provided to the first steam network has a pressure from 1.5 to 11 MPa.

14. The use of a steam compressor in a urea-producing plant according to any one of claims 1 to 10 for reducing the carbon dioxide emissions of the urea-producing plant.
